# EUROPÄISCHE PATENTSCHRIFT

(11) **EP 1 161 175 B1**
(45) Veröffentlichungstag und Bekanntmachung des Hinweises auf die Patenterteilung: **11.05.2005**
(21) Anmeldenummer: 00910682.4
(22) Anmeldetag: 18.02.2000
(51) Int. Cl.: A61B 1/12

(54) **MEDIZINISCHES ODER TECHNISCHES ENDOSKOPISCHES INSTRUMENT**
MEDICAL OR TECHNICAL ENDOSCOPIC INSTRUMENT
INSTRUMENT ENDOSCOPIQUE MEDICAL OU TECHNIQUE

(30) Priorität: 09.03.1999 DE 19910295
(43) Veröffentlichungstag der Anmeldung: 12.12.2001
(73) Patentinhaber: Karl Storz GmbH & Co. KG, 78532 Tuttlingen (DE)
(72) Erfinder: GMINDER, Frank, D-78647 Trossingen (DE); DITTRICH, Horst, D-78194 Immendingen (DE); DOLL, Frank, D-78589 Dürbheim (DE)
(74) Vertreter: Heuckeroth, Volker, Dr.
(86) Internationale Anmeldenummer: PCT/EP2000/001316
(87) Internationale Veröffentlichungsnummer: WO 2000/053079

(56) Entgegenhaltungen:
- EP-A- 0 664 101
- WO-A-92/20274
- WO-A-96/39915
- WO-A-98/43531
- DE-C- 19 520 277
- DE-U- 8 621 769
- FR-A- 1 548 389

## Beschreibung

Die Erfindung betrifft ein medizinisches oder technisches endoskopisches Instrument, mit einem Schaft, in dem eine Endoskopoptik angeordnet ist, die eine distale Stirnfläche aufweist, weiterhin mit einem Arbeitselement, das ebenfalls in dem Schaft angeordnet ist, wobei der Schaft weiterhin als Zuführung einer Spülflüssigkeit in ein Operationsgebiet oder in ein Einsatzgebiet dient, wobei strömungsbeeinflussende Mittel vorgesehen sind, so daß die Spülflüssigkeit vor die Stirnfläche der Endoskopoptik gelangt.

Ein medizinisches Instrument dieser Art ist aus dem Dokument DE-C-195 20 277 bekannt.

Mit einem medizinischen Instrument der eingangs genannten Art wird in der minimal-invasiven Chirurgie unter endoskopischer Sichtkontrolle durch die Endoskopoptik mittels des Arbeitselements Gewebe im menschlichen oder tierischen Körper behandelt, beispielsweise abgetragen, gefaßt und/oder koaguliert.

Zum Behandeln von Gewebe wird beispielsweise ein Arbeitselement verwendet, das zumindest ein Werkzeug aufweist, das als rein mechanisch abtragendes bzw. schneidendes Schneidwerkzeug, beispielsweise in Form von Zangenmaulteilen, oder das als mit Hochfrequenzstrom beaufschlagbare Elektrode ausgebildet ist, mit der unter der Wirkung von Hochfrequenzstrom Gewebe abgetragen bzw. geschnitten oder koaguliert werden kann.

Ein derartiges Instrument für medizinische Zwecke ist beispielsweise aus dem DE-Firmenkatalog der Firma Karl Storz GmbH & Co., Tuttlingen, "Karl Storz - Endoskope", Band Urologie, Seite RES-SC 6 A, Ausgabe 1995, bekannt.

Das aus diesem DE-Firmenprospekt bekannte Instrument ist ein Resektoskop, bei dem verschiedene vorgenannte Arbeitselemente mit Schneidelektroden, Koagulationselektroden oder Küretten in den Schaft einsetzbar sind, mit denen der jeweils vorzunehmende Eingriff ausgeführt werden kann. Die Erfindung ist jedoch nicht auf ein solches Resektoskop beschränkt.

Es sind auch endoskopische Instrumente für technische Zwecke dieser Art bekannt, die zum Arbeiten in schwer zugänglichen Räumen von Maschinen, Motoren oder dgl. verwenden werden.

Da beim Abtragen von Gewebe naturgemäß Blutungen auftreten, ist bei solchen Instrumenten vorgesehen, durch den Schaft eine Spülflüssigkeit in das Operationsgebiet einzuleiten, um die Sicht behinderndes Blut wegzuspülen. Die Spülflüssigkeit wird vom proximalen Ende des Schafts her durch den Schaft zum distalen Ende hin geleitet, wo sie aus dem distalen Ende des Schafts austritt, um das Operationsgebiet im Bereich des Werkzeuges zu spülen. Üblicherweise ist der im Schaft bei eingesetzter Endoskopoptik und bei eingesetztem Arbeitselement noch verbleibende Querschnitt als Spülquerschnitt nutzbar. Die Spülflüssigkeit strömt somit in dem Schaft entlang der Endoskopoptik an dessen Stirnfläche vorbei.

Dabei ergibt sich das Problem, daß sich in der Strömung der Spülflüssigkeit entlang der Endoskopoptik distal vor der Stirnfläche der Endoskopoptik, die das distale lichteintritt- bzw. lichtaustrittseitige Ende der Endoskopoptik bildet, ein Strömungstotraum bildet. Dies bedeutet, daß die Spülflüssigkeit während des Spülens nicht distal vor die Stirnfläche der Endoskopoptik gelangt, sich dort vielmehr Blut und Gewebestücke ansammeln können, die von der Spülflüssigkeit nicht erfaßt und somit nicht weggespült werden können. Das sich vor der Stirnfläche ansammelnde Blut und die Gewebestücke sind jedoch undurchsichtig und behindern somit die Sichtkontrolle durch die Endoskopoptik. Das Problem eines Strömungstotraumes ergibt sich insbesondere bei Endoskopoptiken für Geradeausblick bzw. Schrägvorausblick, deren distale Stirnfläche nahezu senkrecht zur Strömungsrichtung der Spülflüssigkeit steht, d.h. deren Normale der Stirnfläche mit der Strömungsrichtung einen Winkel von 0° bis etwa 20° einschließt.

Bei Instrumenten, die zum Behandeln von Gewebe mit Hochfrequenzstrom arbeiten, tritt zusätzlich das Problem auf, daß sich beim Behandeln von Gewebe mit Hochfrequenzstrom kleine Gasbläschen bilden, die sich in dem Strömungstotraum vor der distalen Stirnfläche der Endoskopoptik ansammeln und an der Stirnfläche anhaften, und die wegen des Strömungstotraums nicht von der Spülflüssigkeit erfaßt und weggespült werden können.

Das aus dem eingangs genannten Dokument DE-C-195 20 277 bekannte endoskopische Instrument weist einen Schaft, eine darin aufgenommene Endoskopoptik und ein ebenfalls darin aufgenommenes Arbeitselement auf. In dem verbleibenden Zwischenraum zwischen dem Schaft, der Endoskopoptik und dem Arbeitselement wird eine Spülflüssigkeit von proximal nach distal geleitet. Als strömungsbeeinflussende Mittel, damit die Spülflüssigkeit vor die Stirnfläche der Endoskopoptik gelangt, ist in dem Schaft eine Öffnung vorgesehen, sowie ein um den Schaft herum beabstandet angeordneter Außenschaft, wobei im Zwischenraum zwischen dem Außenschaft und dem Schaft ein Unterdruck angelegt wird. Durch den Schaft nach distal geleitete Spülflüssigkeit wird durch die Öffnung in den Zwischenraum zwischen dem Außenschaft und dem Schaft angesaugt, wodurch die Spülflüssigkeit an der Stirnfläche der Endoskopoptik vorbeigeleitet wird.

Der Erfindung liegt die Aufgabe zugrunde, ein Instrument der eingangs genannten Art dahingehend weiterzubilden, daß seine Handhabbarkeit verbessert wird.

Erfindungsgemäß wird diese Aufgabe hinsichtlich des eingangs genannten Instruments dadurch gelöst, daß die strömungsbeeinflussenden Mittel am Arbeitselement vorgesehen sind.

Bei dem erfindungsgemäßen Instrument ist demnach vorgesehen, die Strömung der Spülflüssigkeit im Bereich der Stirnfläche durch geeignete Mittel am Arbeitsinstrument so zu beeinflussen, daß zumindest ein Teil der Spülflüssigkeit vor die Stirnfläche gelenkt wird, so daß sich auch in dem sich bei herkömmlichen Instrumenten ausbildenden Strömungstotraum vor der Stirnfläche eine Spülflüssigkeitsbewegung einstellt, die Blut, Gewebestücke und gegebenenfalls Gasbläschen, die sich vor der Stirnfläche ansammeln und die Sicht beeinträchtigen können, von der Spülflüssigkeit erfaßt und weggespült werden. Insbesondere bei Endoskopoptiken, deren Stirnfläche senkrecht oder nahezu senkrecht zur Strömungsrichtung steht, und bei denen sich in verstärktem Maße ein Strömungstotraum ausbilden kann, wird durch die Erfindung ein Ansammeln von undurchsichtigen Flüssigkeiten oder Gewebestücken vermieden. Die strömungsbeeinflussenden Mittel können so ausgebildet und angeordnet sein, daß sie die ansonsten im wesentlichen laminare Strömung im Bereich der Stirnfläche der Endoskopoptik in eine turbulente Strömung umwandeln, und/oder die die Strömungsrichtung der Spülflüssigkeit so umlenken, daß sich eine Strömungskomponente entlang der Stirnfläche der Endoskopoptik ausbildet. In beiden Fällen wird gewährleistet, daß die Stirnfläche ausreichend mit Spülflüssigkeit gespült wird.

Bei der erfindungsgemäßen Ausgestaltung, wonach die strömungsbeeinflussenden Mittel am Arbeitselement selbst vorgesehen sind, ist von Vorteil, daß sich die strömungsbeeinflussenden Mittel so ausbilden und positionieren lassen, daß sie keine Sichtbehinderung darstellen. Da die Arbeitselemente häufig axial relativ zur Endoskopoptik verschiebbar im Schaft aufgenommen sind, eröffnet diese Ausgestaltung die besonders vorteilhafte Möglichkeit, durch Hin und Herschieben des Arbeitselements jeweils unterschiedliche Strömungsverhältnisse einzustellen, um die Spülflüssigkeit effizient vor die Stirnfläche zu lenken.

Die der Erfindung zugrunde liegende Aufgabe wird somit vollkommen gelöst.

In einer bevorzugten Ausgestaltung sind die strömungsbeeinflussenden Mittel als Strömungshindernisse ausgebildet, die eine Verwirbelung der Spülflüssigkeit vor der Stirnfläche der Optik bewirken.

Hierbei ist von Vorteil, daß eine Verwirbelung der Spülflüssigkeit vor der Stirnfläche der Endoskopoptik besonders wirksam eine Durchmischung der Spülflüssigkeit mit dem sich vor der Stirnfläche ansammelndem Blut und eine besonders aggressive Entfernung von anhaftenden Gasblasen oder Gewebestücken bewirkt.

In einer weiteren bevorzugten Ausgestaltung sind die strömungsbeeinflussenden Mittel als strömungsablenkende Mittel ausgebildet, die eine Komponente der Strömung entlang der Stirnfläche der Endoskopoptik erzeugen.

Auch diese Maßnahme ist vorteilhaft geeignet, einen Strömungstotraum vor der Stirnfläche der Endoskopoptik zu vermeiden, indem die Spülflüssigkeit im Bereich der Stirnfläche aus ihrer ursprünglichen im wesentlichen axial gerichteten Strömungsrichtung so umgelenkt wird, daß sich zumindest ein Teil der Strömung entlang der Stirnfläche ausbildet und die Stirnfläche somit wirksam bespült wird.

In einer weiteren bevorzugten Ausgestaltung weisen die strömungsbeeinflussenden Mittel zumindest ein Strömungselement auf, das eine von der Spülflüssigkeit angeströmte, schräg oder quer zur Strömungsrichtung stehende Fläche aufweist.

Die von der Spülflüssigkeit angeströmte, schräg oder quer zur Strömungsrichtung stehende und von dem Arbeitselement abstehen de Fläche führt auf konstruktiv vorteilhaft einfache Weise zu einer Verwirbelung der Spülflüssigkeit im Bereich der Stirnfläche der Endoskopoptik. Ein derartiges Strömungselement oder derartige Strömungselemente können besonders einfach als Anbauteile an dem Arbeitselement angebracht werden, beispielsweise in Form von kleinen Stäben, Plättchen, Leitblechen oder dergleichen.

Dabei ist es weiterhin bevorzugt, wenn die Fläche eben ist oder eine zu einer Endoskopoptikachse hin gerichtete konkave Krümmung aufweist.

Im Falle einer ebenen, schräg oder quer zur Strömungsrichtung stehenden Fläche wirkt diese Fläche im wesentlichen als Prallfläche, die zu einer Verwirbelung der Spülflüssigkeit führt. Durch eine konkav gekrümmte Fläche kann eine Ablenkung der Strömungsrichtung bewirkt werden, so daß die Spülflüssigkeit entlang der Stirnfläche der Endoskopoptik strömt.

In einer weiteren bevorzugten Ausgestaltung sind am Arbeitselement axial und/oder umfänglich verteilt mehrere Strömungselemente angeordnet.

Diese Maßnahme hat den Vorteil, daß eine gezielte Beeinflussung der Strömung der Spülflüssigkeit durch mehrere Strömungselemente, die am Arbeitselement verteilt angeordnet sind, verstärkt werden kann. Weiterhin wird vorteilhaft bei einem axial beweglichen Arbeitselement in mehreren axial unterschiedlichen Arbeitsstellungen des Arbeitselements eine hinreichende Bespülung des Raumes vor der Stirnfläche der Endoskopoptik gewährleistet.

In einer weiteren bevorzugten Ausgestaltung weist das Arbeitselement in einem distalen Bereich einen gegabelten Abschnitt auf, wobei zwei Schenkel des gegabelten Abschnitts seitlich der Endoskopoptik längs derselben verlaufen und in einen proximalen einstrangigen Abschnitt zusammengeführt sind, der sich längs neben der Endoskopoptik erstreckt, und wobei das Arbeitselement im Übergangsbereich von dem gegabelten Abschnitt in den einstrangigen Abschnitt eine Kröpfung aufweist, die in zumindest einer axialen Arbeitsstellung des Arbeitselements axial im Bereich der Stirnfläche der Endoskopoptik liegt.

Bei herkömmlichen Arbeitselementen dieser Art, die üblicherweise als Elektrodenträger für eine mit Hochfrequenzstrom beaufschlagbare Elektrode dienen, ist eine derartige Kröpfung zwar bereits vorgesehen, jedoch befindet sich bei diesen bekannten Arbeitselementen die Kröpfung in jeder axialen Arbeitsstellung des Arbeitselementes relativ zur Endoskopoptik proximal hinter der Stirnfläche der Endoskopoptik. Demgegenüber ist bei der zuvor genannten erfindungsgemäßen Ausgestaltung vorgesehen, die Kröpfung des Arbeitselementes, die sich in dem etwa V-förmigen Übergangsbereich zwischen dem gegabelten Abschnitt und dem einstrangigen Abschnitt befindet, so auszugestalten, daß sich die Kröpfung in zumindest einer Stellung des Arbeitselementes axial im Bereich der Stirnfläche der Endoskopoptik befindet, so daß die Kröpfung vorteilhaft ebenfalls als strömungsbeeinflussende Maßnahme zur gezielten Beeinflussung der Strömung der Spülflüssigkeit im Bereich distal vor der Stirnfläche der Endoskopoptik dienen kann.

In einer weiteren bevorzugten Ausgestaltung ist das Arbeitselement relativ zur Endoskopoptik axial verschiebbar.

Hierbei ist von Vorteil, daß sich die gezielte Beeinflussung der Strömung der Spülflüssigkeit durch ein axiales Hin- und Herbewegen noch zusätzlich verstärken läßt.

In einer weiteren bevorzugten Ausgestaltung ist das Arbeitselement ein Elektrodenträger und das zumindest eine Werkzeug eine mit HF-Strom beaufschlagbare Elektrode.

Wie bereits eingangs erwähnt, tritt bei einem mit Hochfrequenzstrom arbeitenden Instrument das zusätzliche Problem auf, daß sich durch die Wirkung des Hochfrequenzstroms Gasbläschen bilden, die an der Stirnfläche der Endoskopoptik anhaften. Durch die erfindungsgemäß vorgesehenen strömungsbeeinflussenden Mittel wird nunmehr vorteilhaft erreicht, daß auch die Gasbläschen durch gezielte Beeinflussung der Strömung der Spülflüssigkeit von der Stirnfläche der Endoskopoptik weggespült werden können.

Ebenso bevorzugt ist es jedoch, wenn das Arbeitselement einen Schaft mit einem mechanisch wirkenden Werkzeug aufweist.

Auch bei Instrumenten, die nicht mit Hochfrequenzstrom arbeiten, konnte sich bei herkömmlichen Instrumenten Blut vor der Stirnfläche der Endoskopoptik ansammeln, weil sich dort ein Strömungstotraum bildete. Durch die Erfindung wird auch bei rein mechanisch arbeitenden Instrumenten durch eine gezielte Beeinflussung der Strömung der Spülflüssigkeit mittels der strömungsbeeinflussenden Mittel dieses Problem wirksam beseitigt.

Weitere Vorteile ergeben sich aus der nachfolgenden Beschreibung und der beigefügten Zeichnung.

Es versteht sich, daß die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausgewählte Ausführungsbeispiele der Erfindung sind in der Zeichnung dargestellt und werden mit Bezug auf diese hiernach näher beschrieben. Es zeigen:
- Fig. 1: eine Seitenansicht eines endoskopischen Instrumentes in einer Gesamtdarstellung;
- Fig. 2: einen teilweisen Längsschnitt durch den distalen Bereich des Instruments in Fig. 1 in einer Seitenansicht in vergrößertem Maßstab;
- Fig. 3: einen teilweisen Längsschnitt durch den distalen Bereich des Instruments in Fig. 1 in einer Draufsicht in vergrößertem Maßstab;
- Fig. 4: eine Vorderansicht des distalen Endes des Arbeitselementes des Instruments; und
- Fig. 5: einen Fig. 2 entsprechenden teilweisen Längsschnitt durch das Instrument in Fig. 1, wobei das Arbeitselement gemäß Fig. 2 bis 4 durch ein mechanisch arbeitendes Arbeitselement ersetzt ist.

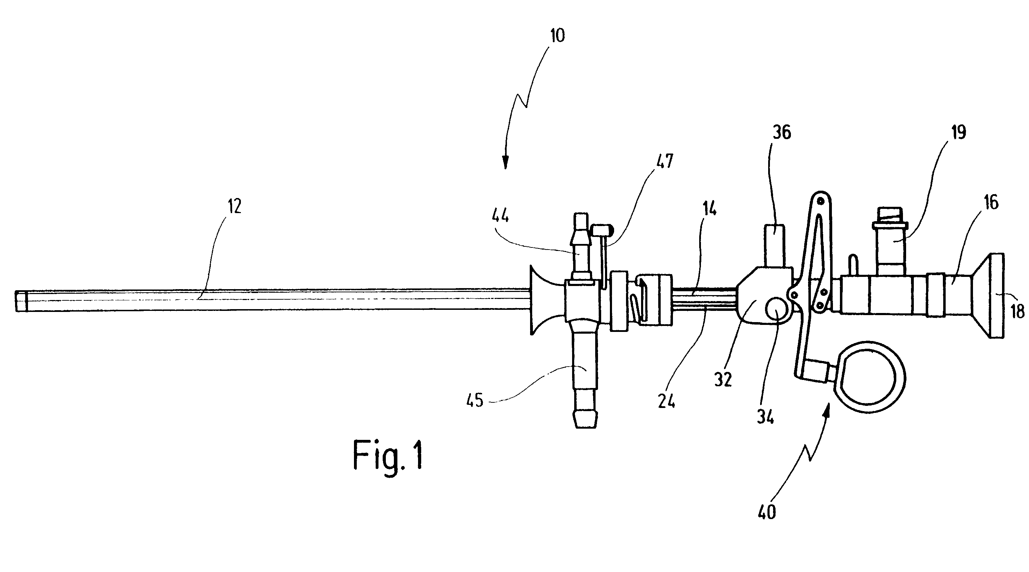

In Fig. 1 bis 4 ist ein mit dem allgemeinen Bezugszeichen 10 versehenes medizinisches endoskopisches Instrument dargestellt. In dem dargestellten Ausführungsbeispiel ist das Instrument 10 ein Resektoskop, das in der Urologie zum minimal-invasiven Abtragen von Gewebe in der Harnblase oder der Prostata verwendet wird.

Die Erfindung wird im folgenden mit Bezug auf dieses medizinische Instrument beschrieben, sie kann jedoch genauso bei technischen endoskopischen Instrumenten eingesetzt werden.

Das Instrument 10 weist einen lang erstreckten Schaft 12 auf. In dem Schaft 12 ist eine Endoskopoptik 14 angeordnet, die an ihrem proximalen Ende ein Okular 16 mit einer Okularmuschel 18 aufweist. Ein Lichtleiteranschluß 19 am Okular 16 dient zum Anschließen eines Lichtleitkabels (nicht dargestellt), um Licht in die Endoskopoptik 14 einzuspeisen.

Von der Endoskopoptik 14 ist in den Fig. 1 bis 3 genauer gesagt der Optikschaft dargestellt, in dem ein bildgebendes System, beispielsweise in Form einer Relaislinsenanordnung und ein Licht zuführendes System in Form von Lichtleitfasern aufgenommen ist, das mit dem Lichtleiteranschluß 19 kommuniziert.

Die Endoskopoptik 14 weist an ihrem distalen Ende eine distale Stirnfläche 20 auf, die das lichtaustritts- bzw. lichteintrittsseitige Ende der Endoskopoptik 14 darstellt. Die Stirnfläche 20 wird durch ein Deckglas gebildet, das den Optikschaft der Endoskopoptik 14 distal verschließt. Die Endoskopoptik 14 ist eine sogenannte Vorausblick-(0°-)Optik, bei der die Stirnfläche 20 senkrecht zu einer Endoskopoptikachse 21 steht.

Die Endoskopoptik 14 ist in dem Schaft 12 in ihrer Arbeitsposition relativ zu diesem unbeweglich angeordnet, wobei die distale Stirnfläche 20 der Endoskopoptik 14 noch im Schaft 12 selbst angeordnet ist und sich proximal hinter einer distalen Öffnung 22 des Schafts 12 befindet. Die Endoskopoptik 14 ist aus dem Schaft 12 zum Zerlegen des Instruments 10 herausnehmbar.

In dem Schaft 12 ist weiterhin ein Arbeitselement 24 angeordnet.

Das Arbeitselement 24 weist an seinem distalen Ende ein Werkzeug 26 in Form einer mit Hochfrequenz-(HF-)Strom beaufschlagbaren Elektrode 28 als Schneidelektrode auf, die hier aus der Längsrichtung des Arbeitselements 24 abgewinkelt ist.

Das übrige Arbeitselement 24 bildet somit einen Elektrodenträger 30, der aus einem isolierten Schaft besteht, in dem eine nicht näher dargestellt Stromzuleitung bis zu der Elektrode 28 führt.

Gemäß Fig. 1 ist ein proximales Ende des Arbeitselements 24 mit einem Gehäuseteil 32 verrastet verbunden, wobei ein Rastknopf 34 zum Lösen des Arbeitselements 24 von dem Gehäuseteil 32 dient. An dem Gehäuseteil 32 befindet sich ein HF-Anschluß 36 zum Anschließen eines nicht dargestellten HF-Kabels, das mit einem HF-Generator verbunden ist.

Das Arbeitselement 24 ist weiterhin relativ zu der Endoskopoptik 14 axial gemäß einem Doppelpfeil 38 hin- und herbewegbar, wobei zum Verschieben des Arbeitselements 24 gemäß Fig. 1 ein Bedienungselement 40 vorgesehen ist, das mit dem ebenfalls axial beweglichen Gehäuseteil 32 verbunden ist.

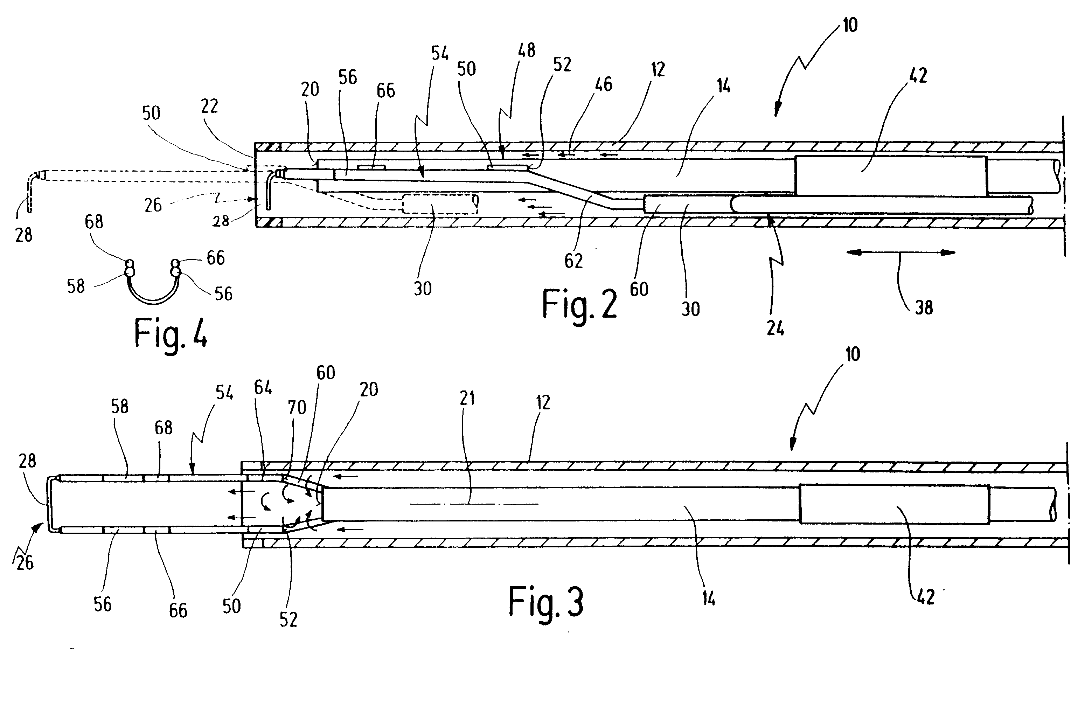

Die axiale Bewegung des Arbeitselements 24 ist dabei über eine Hülse 42 (Fig. 2 und 3), durch die die Endoskopoptik 14 verläuft, entlang der Endoskopoptik 14 geführt.

In Fig. 2 ist das Arbeitselement 24 mit durchgezogenen Linien in seiner maximal zurückgezogenen Stellung dargestellt, in der die Elektrode 28 vor der distalen Stirnfläche 20 der Endoskopoptik 14 zu liegen kommt. Mit unterbrochenen Linien ist die distal maximal vorgeschobene Stellung des Arbeitselements 24 dargestellt.

Der Schaft 12, genauer gesagt der Innenraum des Schafts 12, dient weiterhin als Zuführung einer Spülflüssigkeit, die über einen Spülanschluß 44 zum Anschließen eines Spülschlauchs (nicht dargestellt) in den Schaft 12 an seinem proximalen Ende eingeleitet wird und durch den Innenraum des Schaftes 12 zum distalen Ende hin geleitet wird. Dabei dient der gesamte neben der Endoskopoptik 14 und dem Arbeitselement 24 verbleibende freie Innenquerschnitt des Schafts 12 als Spülquerschnitt. Eine Strömung der Spülflüssigkeit, die sich in dem Schaft 12 axial ausbreitet, ist in Fig. 2 mit Pfeilen 46 dargestellt. Die Spülflüssigkeit wird bis zu der distalen Öffnung 22 des Schafts geführt und tritt dort aus dem Schaft 12 in das zu behandelnde Operationsgebiet aus.

Die Spülflüssigkeit besitzt im vorliegenden Fall, bei dem das Instrument 10 ein HF-Instrument ist, eine geringe Leitfähigkeit.

Die Stirnfläche 20 der Endoskopoptik 14 steht somit senkrecht zur Strömungsrichtung der Spülflüssigkeit. Dem Spülanschluß 44 gegenüberliegend ist ein Absauganschluß 45 angeordnet, an den ein Saugschlauch (nicht dargestellt) anschließbar ist, um die Spülflüssigkeit später wieder aus dem Operationsgebiet abzusaugen. Ein Umschalthebel 47 dient zum Umschalten zwischen Spülen und Saugen.

Um zu vermeiden, daß sich im Bereich unmittelbar vor der distalen Stirnfläche 20 der Endoskopoptik 14 ein Strömungstotraum ausbildet, in dem sich Blut, Gewebereste oder Gasbläschen, die infolge der Behandlung des Gewebes mit Hochfrequenzstrom entstehen, ansammeln können, sind strömungsbeeinflussende Mittel 48 vorgesehen, die bewirken, daß die Stirnfläche 20 bzw. der Raum unmittelbar vor dieser während des Operationsvorganges ausreichend mit Spülflüssigkeit bespült wird, um eine ungehinderte Sichtkontrolle des Operationsvorganges zu ermöglichen.

Die strömungsbeeinflussenden Mittel 48 umfassen in dem in Fig. 1 bis 4 dargestellten Ausführungsbeispiel zumindest ein Strömungselement 50, das eine von der Spülflüssigkeit angeströmte, schräg oder quer zur Strömungsrichtung gemäß den Pfeilen 46 stehende Fläche 52 aufweist. Die Fläche 52 ist eben ausgebildet.

Das Strömungselement 50 ist am Arbeitselement 24 in Form eines Stabes ausgeführt, das als Anbauteil an dem Arbeitselement 24 angebracht ist, beispielsweise durch Löten, Schweißen, Kleben, oder durch Anklemmen, so daß es auch abnehmbar oder lageverstellbar ist.

Das Arbeitselement 24 weist in seinem distalen Bereich einen gegabelten Abschnitt 54 auf, der aus zwei Schenkeln 56 und 58 gebildet wird. Die Schenkel 56 und 58 verlaufen in der in Fig. 2 mit durchgezogenen Linien dargestellten zurückgezogenen Stellung des Arbeitselementes 24 seitlich der Endoskopoptik 14 entlang derselben. Die Schenkel 56 und 58 tragen an ihrem distalen Ende die als Schlinge (Fig. 4) ausgebildete Elektrode 28.

Die Schenkel 56 und 58 sind in einen proximalen einstrangigen Abschnitt 60 des Elektrodenträgers 30 zusammengeführt, der sich längs und außerhalb der Längsachse der Endoskopoptik 14 erstreckt.

Zwischen dem gegabelten Abschnitt 54 und dem einstrangigen Abschnitt 60 des Arbeitselementes 24 weist das Arbeitselement 24 eine Kröpfung 62 auf, die den gegabelten Abschnitt 54 mit dem einstrangigen Abschnitt 60 verbindet, wobei die Schenkel 56 und 58 im Bereich der Kröpfung 62 etwa V-förmig zusammenlaufen. Die Kröpfung 62 ist ebenfalls Teil der strömungsbeeinflussenden Mittel 48.

Die strömungsbeeinflussenden Mittel 48 weisen neben dem zuvor genannten Strömungselement 50 ein weiteres Strömungselement 64 auf, wobei das Strömungselement 50 an dem Schenkel 56 und das Strömungselement 64 an dem Schenkel 58 angeordnet ist, und zwar jeweils am distalen Ende der Kröpfung.

Axial von den Strömungselementen 50 und 64 beabstandet sind weitere Strömungselemente 66 und 68 an dem gegabelten Abschnitt 54 des Arbeitselements 24 angeordnet.

Außer den in Fig. 2 und 3 dargestellten Strömungselementen 50, 64, 66, 68 können noch weitere Strömungselemente axial und/oder umfänglich verteilt an dem Arbeitselement 24, genauer gesagt an den Schenkeln 56 und 58 des gegabelten Abschnitts 54 vorgesehen sein.

Das Strömungselement 50, genauer gesagt die von der Spülflüssigkeit angeströmte Fläche 52, stellt ein Strömungshindernis in der Art einer Prallfläche dar, die eine Verwirbelung der Spülflüssigkeit unmittelbar vor der Stirnfläche 20 der Endoskopoptik bewirkt, wenn sich das Arbeitselement 14 in der in Fig. 2 mit unterbrochenen Linien dargestellten Arbeitsstellung bzw. in der in Fig. 3 dargestellten Arbeitsstellung befindet. Die gleiche Wirkung hat eine von der Spülflüssigkeit angeströmte Fläche 70 des Strömungselements 64.

In der in Fig. 3 dargestellten Arbeitsstellung des Arbeitselements 24 stellt die Kröpfung 62 ebenfalls strömungsbeeinflussende Mittel 48 dar, die zu einer zusätzlichen Verwirbelung der Spülflüssigkeit und damit zu einer Umspülung der Stirnfläche 20 der Endoskopoptik 14 führt. Der Verwirbelungseffekt kann noch dadurch verstärkt werden, daß das Arbeitselement 24 gemäß dem Doppelpfeil 38 hin- und herbewegt wird, wodurch die Strömungsverhältnisse im Bereich der distalen Stirnfläche 20 der Endoskopoptik 14 ebenfalls beeinflußt werden können, daß genügend Spülflüssigkeit vor die Stirnfläche 20 gelenkt wird.

Wenn das Arbeitselement 24 aus der in Fig. 3 dargestellten maximal distalen Arbeitsstellung soweit nach proximal zurückgezogen wird, so daß die Strömungselemente 66 und 68 axial vor der Stirnfläche 20 zu liegen kommen, tritt eine Strömungsbeeinflussung aufgrund dieser Strömungselemente 66 und 68, wie zuvor beschrieben auf.

Während die Strömungselemente 50, 64, 66 und 68 als kleine Stäbe ausgebildet sind, die an dem Arbeitselement 24 befestigt sind, können die strömungsbeeinflussenden Mittel auch in Form von Plättchen, Leitblechen, Noppen oder Leitschaufeln oder dergleichen ausgebildet sein. Solche Strömungselemente können als Anbauteile am Arbeitselement 24 lösbar oder unlösbar befestigt oder mit diesem einstückig ausgebildet sein. Es können auch Strömungselemente verwendet werden, die eine von der Spülflüssigkeit angeströmte, zu der Endoskopoptikachse 21 hin konkav gekrümmte Fläche aufweisen, so daß die strömungsbeeinflussende Wirkung im wesentlichen in einer Richtungsänderung der Strömungsrichtung eher als in einer Verwirbelung besteht.

Ferner können derartige Strömungselemente auch am Schaft 12 oder an der Endoskopoptik 14 im Bereich der distalen Stirnfläche 20 angeordnet sein, um die Strömung der Spülflüssigkeit geeignet zu beeinflussen, so daß Verunreinigungen vor der Stirnfläche 20 weggespült werden können.

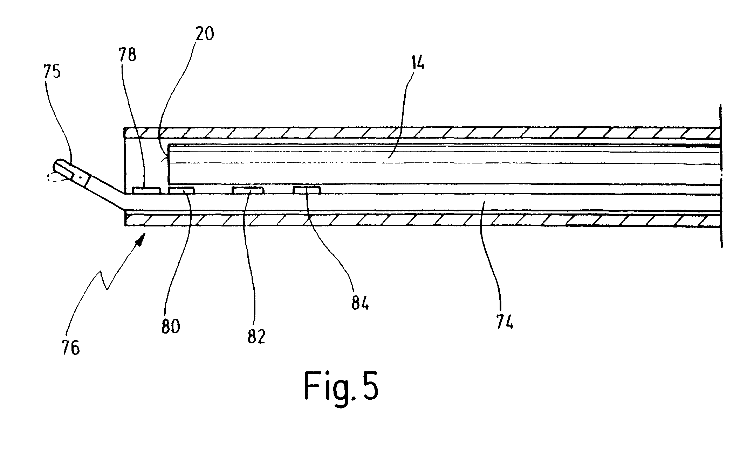

Während bei dem Ausführungsbeispiel gemäß Fig. 1 bis 4 das Arbeitselement 24 ein Arbeitselement zum Abtragen von Gewebe mittels Hochfrequenzstrom ist, wobei anstelle einer Schneidelektrode wie der Elektrode 28 auch eine Koagulationselektrode an dem Arbeitselement 24 vorhanden sein kann, kann die Erfindung auch bei einem in Fig. 5 dargestellten Arbeitselement 74 eingesetzt werden, das an seinem distalen Ende ein rein mechanisch wirkendes Werkzeug 75 in Form von Zangenmaulteilen aufweist. Auch das Arbeitselement 74 weist strömungsbeeinflussende Mittel 76 auf, die ein oder mehrere Strömungselement 78 bis 84 umfassen, die vor der distalen Stirnfläche 20 der Endoskopoptik 14 je nach der axialen Arbeitsstellung des Arbeitselementes 74 bewirken, daß die Spülflüssigkeit vor die distale Stirnfläche 20 gelangt.

## Patentansprüche

1. Medizinisches oder technisches endoskopisches Instrument, mit einem Schaft (12), in dem eine Endoskopoptik (14) angeordnet ist, die eine distale Stirnfläche (20) aufweist, weiterhin mit einem Arbeitselement (24; 74), das ebenfalls in dem Schaft (12) angeordnet ist, wobei der Schaft (12) weiterhin als Zuführung einer Spülflüssigkeit in ein Operationsgebiet oder in ein Einsatzgebiet dient, wobei strömungsbeeinflussende Mittel (48; 76) vorgesehen sind, so daß die Spülflüssigkeit vor die Stirnfläche (20) der Endoskopoptik (14) gelangt, **dadurch gekennzeichnet, daß** die strömungsbeeinflussenden Mittel am Arbeitselement (24; 74) vorgesehen sind.

2. Instrument nach Anspruch 1, **dadurch gekennzeichnet, daß** die strömungsbeeinflussenden Mittel (48; 76) als Strömungshindernisse ausgebildet sind, die eine Verwirbelung der Spülflüssigkeit vor der Stirnfläche (20) der Endoskopoptik (14) bewirken.

3. Instrument nach Anspruch 1 oder 2, **dadurch gekennzeichnet, daß** die strömungsbeeinflussenden Mittel (48; 76) als strömungsablenkende Mittel ausgebildet sind, die eine Komponente der Strömung entlang der Stirnfläche (20) der Endoskopoptik (14) erzeugen.

4. Instrument nach einem der Ansprüche 1 bis 3, **dadurch gekennzeichnet, daß** die strömungsbeeinflussenden Mittel (48; 76) zumindest ein Strömungselement (50; 64, 66, 68; 78; 80, 82, 84) aufweisen, das eine von der Spülflüssigkeit angeströmte, schräg oder quer zur Strömungsrichtung stehende Fläche (52, 70) aufweist.

5. Instrument nach Anspruch 4, **dadurch gekennzeichnet, daß** die Fläche (52, 70) eben ist oder eine zu einer Endoskopoptikachse (21) hin gerichte konkave Krümmung aufweist.

6. Instrument nach Anspruch 4 oder 5, **dadurch gekennzeichnet, daß** am Arbeitselement (24; 74) axial und/oder umfänglich verteilt mehrere Strömungselemente (50; 64, 66, 68; 78; 80, 82, 84) angeordnet sind.

7. Instrument nach einem der Ansprüche 1 bis 6, **dadurch gekennzeichnet, daß** das Arbeitselement (24) in einem distalen Bereich einen gegabelten Abschnitt (54) aufweist, wobei zwei Schenkel (56, 58) des gegabelten Abschnitts (54) seitlich der Endoskopoptik (14) längs derselben verlaufen und in einen proximalen einstrangigen Abschnitt (60) zusammengeführt sind, der sich längs neben der Endoskopoptik (14) erstreckt, und wobei das Arbeitselement (14) im Übergangsbereich von dem gegabelten Abschnitt (54) in den einstrangigen Abschnitt (60) eine Kröpfung (62) aufweist, die in zumindest einer axialen Arbeitsstellung des Arbeitselements (24) axial im Bereich der Stirnfläche (20) der Endoskopoptik (14) liegt.

8. Instrument nach einem der Ansprüche 1 bis 7, **dadurch gekennzeichnet, daß** das Arbeitselement (24; 74) relativ zur Endoskopoptik (14) axial verschiebbar ist.

9. Instrument nach einem der Ansprüche 1 bis 8, **dadurch gekennzeichnet, daß** das Arbeitselement (24) ein Elektrodenträger (30) ist, der eine mit HF-Strom beaufschlagbare Elektrode (28) trägt.

10. Instrument nach einem der Ansprüche 1 bis 9, **dadurch gekennzeichnet, daß** das Arbeitselement (74) einen Schaft mit einem mechanisch wirkenden Werkzeug (75) aufweist.

## Claims

1. A medical or technical endoscopic instrument, comprising a shaft (12) having an endoscope optical system (14) arranged therein, which comprises a distal front face (20), and a working element (24, 74) also arranged in the shaft (12), wherein, further, the shaft (12) serves to supply an irrigation fluid into an operation or application area, wherein flow-influencing means (48; 76) are provided such that the irrigation fluid reaches in front of the front face (20) of the endoscope optical system (14), **characterized in that** the flow-influencing means are provided on the working element (24; 74).

2. The instrument of claim 1, **characterized in that** the flow-influencing means (48; 76) are configured as flow obstructions which cause a swirling of the irrigation fluid in front of the front face (20) of the endoscope optical system (14).

3. The instrument of claim 1 or 2, **characterized in that** the flow-influencing means (48; 76) are configured as flow deflection means, which generate a flow component along the front face (20) of the endoscope optical system (14).

4. The instrument of anyone of claims 1 through 3, **characterized in that** the flow-influencing means (48; 76) comprise at least one flow element (50; 64, 66, 68; 78; 80, 82, 84) which has a surface (52, 70), against which the irrigation fluid flows and which is inclined or runs transversely with respect to the flow direction.

5. The instrument of claim 4, **characterized in that** the surface (52, 70) is flat or has a concave curvature directed to an axis (21) of the endoscope optical system.

6. The instrument of claim 4 or 5, **characterized in that** several flow elements (50, 64, 66, 68; 78; 80, 82, 84) are arranged axially and/or circumferentially distributed on the working element (24; 74).

7. The instrument of anyone of claims 1 through 6, **characterized in that** the working element (24) comprises a forked section (54) in a distal region, wherein two legs (56, 58) of the forked section (54) run axially along the sides of the endoscope optical system (14) and run together in a proximal unitary section (60), which extends axially adjacent to the endoscope optical system (14), and wherein the working element (24) comprises a bend (62) in the transition region from the forked section (54) to the unitary section (60), which lies axially in the region of the front face (20) of the endoscope optical system (14) in at least one axial working position of the working element (24).

8. The instrument of anyone of the claims 1 through 7, **characterized in that** the working element (24; 74) is axially shiftable relative to the endoscope optical system (14).

9. The instrument of anyone of claims 1 through 8, **characterized in that** the working element (24) is an electrode carrier (30) which carries an electrode (28) supplyable with HF-current.

10. The instrument of anyone of claims 1 through 9, **characterized in that** the working element (74) comprises a shaft with a mechanically acting tool (75).

## Revendications

1. Instrument endoscopique médical ou technique avec un tube (12) dans lequel une optique d'endoscope (14) est disposée, qui comporte une surface frontale distale (20), de plus avec un élément de travail (24, 74) qui est également disposé dans le tube (12), le tube (12) servant de plus à amener un liquide de rinçage dans un champ opératoire ou dans une zone d'utilisation, des agents influant sur l'écoulement (48, 76) étant prévus, de manière à ce que le liquide de rinçage arrive devant la surface frontale (20) de l'optique de l'endoscope (14), **caractérisé en ce que** les agents influant sur l'écoulement sont prévus sur l'élément de travail (24, 74).

2. Instrument selon la revendication 1, **caractérisé en ce que** les agents influant sur l'écoulement (48, 76) sont réalisés comme des obstacles à l'écoulement qui provoquent un tourbillonnement du liquide de rinçage devant la surface frontale (20) de l'optique de l'endoscope (14).

3. Instrument selon la revendication 1 ou 2, **caractérisé en ce que** les agents influant sur l'écoulement (48, 76) sont réalisés comme agents de déviation de l'écoulement qui génèrent une composante de l'écoulement le long de la surface frontale (20) de l'optique de l'endoscope (14).

4. Instrument selon une des revendications 1 à 3, **caractérisé en ce que** les agents influant sur l'écoulement (48, 76) comportent au moins un élément d'écoulement (50, 64, 66, 68, 78, 80, 82, 84) qui comporte une surface oblique ou transversale au sens d'écoulement (52, 70) sur laquelle le liquide de rinçage s'écoule.

5. Instrument selon la revendication 4, **caractérisé en ce que** la surface (52, 70) est plane ou comporte une courbe concave dirigée vers un axe de l'optique de l'endoscope (21).

6. Instrument selon la revendication 4 ou 5, **caractérisé en ce que** plusieurs éléments d'écoulement (50, 64, 66, 68, 78, 80, 82, 84) répartis axialement et/ou sur la circonférence sont disposés sur l'élément de travail (24, 74).

7. Instrument selon une des revendications 1 à 6, **caractérisé en ce que** l'élément de travail (24) comporte dans une zone distale une section ramifiée (54), deux branches (56, 58) de la section ramifiée (54) passant latéralement par rapport à l'optique de l'endoscope (14) longitudinalement par rapport à cette dernière et étant réunies dans une section proximale à une barre (60) qui s'étend longitudinalement près de l'optique de l'endoscope (14), et l'élément de travail (24) comportant dans la zone de transition de la section ramifiée (54) vers la section à une barre (60) un coude (62) qui se situe dans au moins une position de travail axiale de l'élément de travail (24) axialement dans la zone de la surface frontale (20) de l'optique de l'endoscope (14).

8. Instrument selon une des revendications 1 à 7, **caractérisé en ce que** l'élément de travail (24, 74) peut être déplacé axialement par rapport à l'optique de l'endoscope (14).

9. Instrument selon une des revendications 1 à 8, **caractérisé en ce que** l'élément de travail (24) est un support d'électrode (30) qui porte une électrode (28) à laquelle un courant HF peut être appliqué.

10. Instrument selon une des revendications 1 à 9, **caractérisé en ce que** l'élément de travail (24) comporte un tube avec un outil à effet mécanique (75).
